# EUROPEAN PATENT SPECIFICATION

(11) **EP 2 747 758 B1**
(45) Date of publication and mention of the grant of the patent: **30.03.2016**
(21) Application number: 12766319.3
(22) Date of filing: 21.08.2012
(51) Int. Cl.: A61K 31/135, A61K 31/137, A61K 31/165, A61K 31/27, A61K 31/4045, A61K 31/407, A61K 31/465, A61K 31/473, A61K 47/02, A61K 47/14, A61K 47/38, A61K 9/70

(54) **A DEVICE FOR THE TRANSDERMAL DELIVERY OF ALKALINE COMPOUNDS THAT ARE SUSCEPTIBLE TO DEGRADATION IN THEIR FREE BASE FORM**
VORRICHTUNG ZUR TRANSDERMALEN FREISETZUNG VON IN IHRER FREIEN BASENFORM ABBAUSENSITIVEN ALKALISCHEN VERBINDUNGEN
DISPOSITIF D'ADMINISTRATION TRANSDERMIQUE DE COMPOSÉS ALCALINS QUI SONT SUSCEPTIBLES DE DÉGRADATION SOUS LEUR FORME DE BASE LIBRE

(30) Priority: 25.08.2011 AR P110103098
(43) Date of publication of application: 02.07.2014
(73) Proprietor: Amarin Technologies S.A., Ciudad Autónoma de Buenos Aires 1416 (AR)
(72) Inventor: SCASSO, Alejandro Fabio, Buenos Aires 1852 (AR); STEFANO, Francisco José Evaristo, Ciudad Autónoma de Buenos Aires 1425 (AR)
(74) Representative: Smith, Matthew
(86) International application number: PCT/GB2012/052047
(87) International publication number: WO 2013/027052

(56) References cited:
- US-A1- 2010 087 768
- K. C. GARALA ET AL: "FORMULATION AND IN-VITRO CHARACTERIZATION OF MONOLITHIC MATRIX TRANSDERMAL SYSTEMS USING HPMC/EUDRAGIT S 100 POLYMER BLENDS", INTERNATIONAL JOURNAL OF PHARMACY AND PHARMACEUTICAL SCIENCES, vol. 1, no. Suppl. 1, 31 December 2009 (2009-12-31), pages 108-120, XP055044083,

## Description

The present invention refers to a device for the transdermal delivery of an alkaline pharmaceutically active compound that is susceptible to degradation in its free base form that is rivastigmine, comprising an adhesive matrix layer, a backing layer and a release or protective layer, wherein the adhesive matrix layer comprises said pharmaceutically active compound, an amount of triethylcitrate of between 0.2% and 10% and an amount of hydrochloric acid (HCl) of between 0.05% and 5%. Devices for the transdermal delivery of a compound chosen from the group comprising rivastigmine, selegiline, rasagiline, ropinirole and asenapine are described

### BACKGROUND FOR THE INVENTION

Transdermal delivery of drugs has important advantages over other delivery routes. Among some of said advantages one can include its comfort, its capacity to release the active compound in a controlled and predictable manner, as well as the possibility to quickly interrupt drug release if any adverse effect takes place (by removing the device from the user's skin). Moreover, it allows an improvement in the compliance of therapeutical programs and a reduction in some adverse effects related to the oral delivery of certain drugs.

Using transdermal delivery devices (TDDs) one can achieve the systemic delivery of several active compounds directly through the skin. The earliest devices approved by the Food and Drug Administration (FDA), were the ones containing scopolamine, for the prevention and relieving of kinetosis. Years later, other TDDs were approved for the treatment of several pathologies, including devices for the transdermal delivery of hormones, pain killers, non-steroidal antiinflammatory drugs, nitroglycerin and fentanyl.

Although the devices mentioned above show many advantages, not every drug has been able to be successfully included in this kind of devices for its transdermal delivery.

For example, TDDs containing steroid hormones exhibit problems in their physical stability, so diverse methods have been proposed to avoid the crystalization of active compounds (for examples, refer to US 6,465,005 and US 5,676,968).

On the other hand, drugs that are liquid at a temperature close to room temperature show several problems when included in TDDs. One of said problems is the partial loss of drug during the drying step of the manufacture of the devices, which is usually performed at high temperatures. For example, in application US 2010087768 (A1) it is revealed that squalene or triethylcitrate (TEC) can be used to avoid the problem mentioned above for the transdermal delivery of rivastigmine or selegiline.
Finally, in international application WO 9934782 the use of antioxidants is proposed in order to stabilize the compound rivastigmine in TDDs.

### BRIEF DESCRIPTION OF THE INVENTION

The present invention refers to a device for the transdermal delivery of rivastigmine that comprises an adhesive matrix layer, a backing layer and a release or protective layer, wherein the adhesive matrix layer comprises said rivastigmine, an amount of triethylcitrate of between 0.2% and 10% and an amount of hydrochloric acid (HCl) of between 0.05% and 5%. The device described in the invention complies with the expected pharmacokinetic parameters, is stable during long time periods and, also, exhibits adequate adhesive properties for its use.

### DETAILED DESCRIPTION OF THE INVENTION

The present inventors have developed a novel device for the delivery of rivastigmine that solves the problems found in the prior art. Some alkaline pharmaceutically active compounds that are susceptible to degradation in their free base form are rivastigmine, selegiline, rasagiline, nicotine, apomorphine, agomelatine, ropinirol, and asenapine

It is known that several of these drugs tend to be unstable towards oxidation when they are formulated in pharmaceutical composition in their free base form. The solution an expert in the art would seek to solve this problem would be to add an adequate amount of antioxidants to the composition. In fact, this is the solution proposed in international application WO 9934782, in which it is revealed a rivastigmine formulation comprising rivastigmine in its free base or addition salt forms and an antioxidant.

Surprisingly, the present inventors have found that, regarding TDDs comprising alkaline pharmaceutically active compounds that are susceptible to degradation in their free base form, the simple addition of antioxidant substances is not enough to stabilize said active compounds. As it can be noted in Tables 1 and 2 included below, the simple addition of antioxidants to adhesive formulations containing rivastigmine free base is not enough to stabilize the active compound. This fact is shown by the rivastigmine-related impurities generated while storing the formulations during 6 months at 40 °C and 75% R. H.

**Table 1**

| Lot | 043 | 046 | 047 | 048 | 051 | 052 | 053 | 054 |
|---|---|---|---|---|---|---|---|---|
| Rivastigmine free base | 30.0 | 30.0 | 30.0 | 30.0 | 30.0 | 30.0 | 30.0 | 30.0 |
| Duro-Tak™ 87-2353 | 47.6 | 47.25 | 47.25 | 47.25 | 46.2 | 47.46 | 47.46 | 47.25 |
| Triethylcitrate | 2.0 | 2.0 | 2.0 | 2.0 | 2.0 | 2.0 | 2.0 | 2.0 |
| Ethylcellulose | 20.4 | 20.25 | 20.25 | 20.25 | 19.8 | 20.34 | 20.34 | 20.25 |
| Thiogiycerol | --- | 0.5 | --- | --- | --- | --- | --- | --- |
| Didodecyl Dithiopropionate | --- | --- | 0.5 | --- | --- | --- | --- | --- |
| Sorbic acid | --- | --- | --- | 0.5 | --- | --- | --- | --- |
| Succinic acid | --- | --- | --- | --- | 2.0 | --- | --- | --- |
| Sodium hydroxide 1N | --- | --- | --- | --- | --- | 0.2 | --- | --- |
| Hydrochloric acid 1N | --- | --- | --- | --- | --- | --- | 0.2 | --- |
| α tocopherol | --- | --- | --- | --- | --- | --- | --- | 0.5 |

**Table 2: Impurities determined after 6 storage months at 40 º C and 75% R. H.**

| Lot | Impurities relative retention time | Impurities area/area percent |
|---|---|---|
| 043 | 1.81 | 0.2 % |
| | 2.98 | 0.5 % |
| | Total | 0.7 % |
| 046 | 1.81 | 0.4 % |
| | 2.98 | 0.1 % |
| | Total | 1.5 % |
| 047 | 1.81 | 0.2 % |
| | 2.98 | 0.5 % |
| | Total | 0.7 % |
| 048 | 1.82 | 0.2 % |
| | 2.99 | 0.6 % |
| | Total | 1.0 % |
| 051 | 1.81 | 0.2 % |
| | 2.98 | 0.8 % |
| | Total | 1.2 % |
| 052 | 1.81 | 0.3 % |
| | 2.98 | 0.6 % |
| | Total | 0.9 % |
| 053 | 1.81 | ≤ 0.1 % |
| | 2.98 | ≤ 0.1 % |
| | Total | ≤ 0.1 % |
| 054 | 1.80 | ≤ 0.1 % |
| | 2.98 | 0.5 % |
| | Total | 0.5 % |

Surprisingly, the present inventors have found that TDDs for the delivery of rivastigmine that contain a combination of triethylcitrate and hydrochloric acid in certain proportions (see lot 053 in table 1) are stable and exhibit all the desired pharmaceutical and pharmacokinetic properties. A formulation with these characteristics is not obtained with the addition of triethylcitrate or hydrochloric acid individually. Thus, the present invention refers to a device for the transdermal delivery of rivastigmine that comprises an adhesive matrix layer, a backing layer and a release or protective liner, wherein the adhesive matrix layer comprises said rivastigmine, an amount of triethylcitrate of between 0.2% and 10% and an amount of HCl of between 0.05% and 5%. The device described in the invention meets the expected pharmacokinetic parameters, is stable for long time periods and, moreover, possesses adequate adhesive properties for its use.

It is described a device for the transdermal delivery of a compound selected from the group formed by rivastigmine, selegiline, rasagiline, nicotine, apomorphine, agomelatine, ropinirole and asenapine, comprising an adhesive matrix layer, a backing layer and a release or protective liner, wherein the adhesive matrix layer comprises, at least, one of said pharmaceutically active compounds, an amount of triethylcitrate of between 0.2% and 10% and an amount of HCl of between 0.05% and 5%.

Preferably, HCl is added to the formulation as an ethanolic solution with 1 N concentration. Expressed as added HCl mass, HCl concentration in the composition is between 0.05% and 5%, preferably between about 0.1% and about 2%. During the drying of the device the ethanol is eliminated completely. In the preferred conditions according to the present invention, if one is to consider the equivalents of each compound, the HCl is present in an amount such that it could ionize the drug at as far as 50%. Unless it is specifically stated otherwise, HCl concentration is expressed as HCl mass in relation to the total weight of the adhesive matrix before it is subjected to the drying phase.

In a particular embodiment, the adhesive matrix also comprises ethylcellulose in an amount of between about 10% and about 40%.

It is described a device comprising in its adhesive matrix a pharmaceutically active compound selected from the group defined by rivastigmine, selegiline, rasagiline, nicotine, apomorphine, agomelatine, ropinirole and asenapine, and also comprising an amount of triethylcitrate of between about 1% and about 5% and an amount of HCl of between about 0.1% and about 2%.

In a particularly preferable embodiment of this invention, it is disclosed a device for the transdermal delivery of rivastigmine that comprises an adhesive matrix layer, a backing layer and a release or protective liner, wherein the adhesive matrix layer comprises an amount of triethylcitrate of between 0.2% and 10%, an amount of HCl of between 0.05% and 5%, and an amount of ethylcellulose of between 10% and 40%.

More preferably, the device of the present invention comprises in its adhesive matrix layer a therapeutically effective amount of rivastigmine, and it also comprises an amount of triethylcitrate of between about 1% and about 5%, an amount of HCl of between about 0.1% and about 2%, and an amount of ethylcellulose of between about 20% and about 30%.

It is also described a device for the transdermal delivery of selegiline that comprises an adhesive matrix layer, a backing layer and a release or protective liner, wherein the adhesive matrix layer comprises a therapeutically effective amount of selegiline, an amount of triethylcitrate of between about 0.2% and about 10%, an amount of HCl of between about 0.05% and about 5%.

More preferably, the device comprises in its adhesive matrix layer a therapeutically effective amount of selegiline, and it also comprises an amount of triethylcitrate of between about 1% and about 5% and an amount of HCl of between about 0.1% and about 2%.

The present invention provides a method for preparing a device for the transdermal delivery of rivastigmine comprising:
a) preparing a solution containing said rivastigmine, a polymeric adhesive, triethylcitrate and hydrochloric acid; b) pouring said solution on a release or protective liner so as to form a film that covers the liner; c) drying said film at an appropriate temperature to obtain an adhesive matrix; and d) attaching a backing layer to the adhesive matrix; wherein the adhesive matrix obtained comprises an amount of triethylcitrate of between 0.2% and 10%, and an amount of hydrochloric acid of between 0.05% and 5%.

In some embodiments, and as mentioned above, the hydrochloric acid is added to the initial solution as a 1 N ethanolic solution before adding the alkaline pharmaceutically active compound.

Unless specified otherwise, all percentages are expressed as ingredient mass related to the total weight of the adhesive matrix.

Unless specified otherwise, the adhesive matrix could comprise one or more polymers or copolymers selected from the group defined by polyacrylates, silicone polymers, polyisobutylenes, and block rubber copolymers such as styrene-isoprene-styrene or styrene-butyrene-styrene copolymers. In a preferred embodiment of the invention, the adhesive matrix comprises a polyacrylate, more preferably a polyacrylate with hydroxylic or carboxylic functionality.

As used in the present invention, an "alkaline pharmaceutically active that is susceptible to degradation in its free base form" refers to a compound capable of neutralizing acids and their effects, that is not forming a salt and that possesses a desired pharmacological activity. Some examples for said compounds comprise, but are not limited to, rivastigmine, selegiline, rasagiline, nicotine, apomorphine, agomelatine, ropinirole and asenapine. Additional information about these pharmaceutically acceptable compounds can be found Remington's Pharmaceutical Sciences, 17th ed., Mack Publishing Company, Easton, PA, 1985, or in S. M. Berge, y col., "Pharmaceutical Salts," J. Pharm. Sci., 1977; 66:119.

According to the present invention, a "therapeutically effective amount" of a certain compound is an amount of said compound that, when administered to a patient, effectively treats the corresponding disease. The amount of a certain compound that constitutes a "therapeutically effective amount" may vary depending on several factors, such as the compound's activity, metabolic stability, excretion rate and action durability, the patient's age, weight, general healthiness, gender, diet and species, the simultaneous administration of adjuvants or additional therapies and the severity of the disease for which the therapeutical effect is sought. The therapeutically effective amount for a certain circumstance can usually be determined without need of additional experimentation.

In the case of compounds such as rivastigmine, selegiline, rasagiline, nicotine, apomorphine, agomelatine, ropinirole and asenapine, it is preferred to use an amount comprised between 2% and 30% relative to the total weight of the adhesive matrix. Particularly, for rivastigmine and selegiline it is preferred to use an amount comprised between 10% and 30% relative to the total weight of the adhesive matrix. Preferably, the device disclosed in the present invention comprises 25% of rivastigmine

In international application WO 9934782 it is clearly mentioned that rivastigmine is susceptible to degradation in the presence of oxygen. Regarding this fact, its applicant proposes adding an antioxidant to the composition, presumably achieving a decrease in the degradation of rivastigmine, thus obtaining a lower amount of degradation products. Thus, the triethylcitrate can not be considered as an antioxidant that can be used according to the teachings of application WO 9934782. In fact, neither triethylcitrate nor its hydrolysis product citric acid protects rivastigmine from degradation. As seen in Table 1 shown above, triethylcitrate does not stabilize rivastigmine (see results obtained for lots 043, 046, 047, 048, 051, 052 and 053).

It has been proposed that triethylcitrate can not, by itself, form complex ions with the metallic ions that can catalyze oxidative reactions. This is due to the fact that TEC does not have free carboxyl groups. It can not be hydrolyzed to citric acid either, since there is no water in the composition (or it is present in a negligible concentration).

Thus, according to the argumentation shown above, it is surprising to see a stabilization of the devices disclosed in the invention achieved by the combination of triethylcitrate and HCl in the concentrations proposed in the present invention. The fact that several drugs which, although coinciding in having an alkaline functionality, belong to different families of chemical compounds are stabilized by a combination of TEC and HCl leads one to think that said compounds modify the adhesive matrix in a way that tends to stabilize alkaline drugs. The inventors propose, without pretending to be tied up to any particular hypothesis, that the presence of HCl, bonding with the hydrophobic adhesive polymer by means of a TEC-mediated electrostatic interaction, generates an increase in the polarity of the adhesive matrix which stabilizes the amino group present in the alkaline pharmaceutically active compounds

Besides the adhesives, liners and excipients explicitly mentioned in the present disclosure, those skilled in the art know to choose materials commonly used in the production of devices for the transdermal delivery of drugs, such as permeation enhancers, adhesion enhancers, crystallization inhibitors, etc., that can be included in the device disclosed in the present invention.

Among the examples of crystallization inhibitors one can include, without limiting oneself to, polyvinylpyrrolidone, polyvinyl alcohol, carbomers, aluminum and magnesium silicate, N-methyl-2-pyrrolidone, etc. Among the permeation enhancers one can include, without limiting oneself to, fatty acids and esters, essential oils, pyrrolidones, sulfoxides, alcohols, glycols, cyclodextrines, etc. Among the adhesion enhancers one can include, without limiting oneself to, low molecular weight polyisobutylenes, tackifying resins, etc.

### BRIEF DESCRIPTION OF DRAWINGS

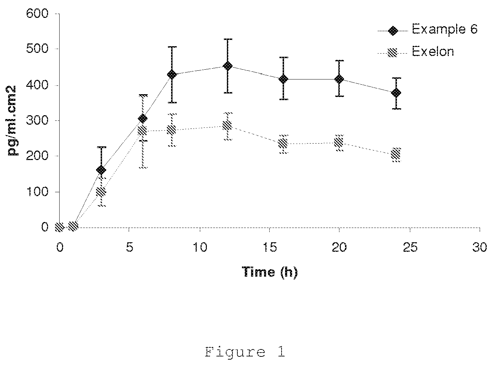

Figure 1 shows a graph of the results of a comparison of plasma concentration profile (expressed by cm² of applied patch) between a patch according to an example of the present invention and a patch currently on the market (Exelon™).

### EXAMPLES

The following Examples describe transdermal devices containing an alkaline pharmaceutically active compound that is susceptible to degradation when it is in its free base form. Transdermal devices containing rivastigmine, selegiline, rasagiline, nicotine, apomorphine, agomelatine, ropinirole and asenapine are exemplified.

### 1) Rivastigmine (according to the invention)

Transdermal devices were prepared by techniques well known for those skilled in the art, in which the adhesive matrixes have the compositions described in Table 3. Duro-Tak® adhesives consist in polyacrylates and are marketed by Henkel AG. Duro-Tak® 87-4287 has a hydroxylic functionality and is not crosslinked.

**Table 3**

| Example | 1 | 2 | 3 | 4 | 5 | 6 | 7 | 8 | 9 | 10 | 11 | 12 |
|---|---|---|---|---|---|---|---|---|---|---|---|---|
| Rivastigmine base | 30.0 | 30.0 | 30.0 | 30.0 | 30.0 | 25.0 | 25.0 | 25.0 | 30.0 | 30.0 | 30.0 | 30.0 |
| Duro-Tak® 87-4287 | 47.6 | 47.46 | 48.9 | 38.0 | 30.0 | 47.79 | 49.54 | 49.27 | 37.75 | 37.5 | 37.25 | 37.0 |
| Triethylcitrate | 2.0 | 2.0 | --- | --- | 10.0 | 2.0 | 0.25 | 0.53 | 2.0 | 2.0 | 2.0 | 2.0 |
| Ethylcellulose | 20.4 | 20.34 | 20.9 | 30.0 | 30.0 | 25.0 | 25.0 | 25.0 | 30.0 | 30.0 | 30.0 | 30.0 |
| Hydrochloric acid | --- | 0.2 | 0.2 | 2.0 | --- | 0.21 | 0.21 | 0.21 | 0.25 | 0.5 | 0.75 | 1.0 |

After subjecting the devices described above to an accelerated stability study (at 40 °C and a relative humidity of 75%) during 6 months, the results showed in Table 4 were obtained.

**Table 4**

| Example | Relative retention time | % w/w |
|---|---|---|
| 1 | 1.81 | 0.3% |
| | 2.98 | 0.3% |
| | Total | 0.6% |
| 2 | 1.81 | ≤ 0.1% |
| | 2.97 | ≤ 0.1% |
| | Total | ≤ 0.1% |
| 3 | 1.81 | 0.3% |
| | 2.97 | 0.2% |
| | Total | 0.5% |
| 6 | 1.81 | ≤ 0.1% |
| | 2.98 | ≤ 0.1% |
| | Total | ≤ 0.1% |
| 7 | 1.81 | 0.2% |
| | 2.98 | ≤ 0.1% |
| | Total | 0.2% |
| 8 | 1.81 | 0.2% |
| | 2.98 | ≤ 0.1% |
| | Total | 0.2% |
| 9 | 1.81 | ≤ 0.1% |
| | 2.98 | 0.2% |
| | Total | 0.2% |
| 10 | 1.81 | ≤ 0.1% |
| | 2.98 | 0.2% |
| | Total | 0.2% |
| 11 | 1.81 | ≤ 0.1% |
| | 2.98 | 0.2% |
| | Total | 0.2% |
| 12 | 1.81 | ≤ 0.1% |
| | 2.98 | 0.2% |
| | Total | 0.2% |

Example 4 exhibits a defective matrix, which is not appropriate to se used as a TDS. This matrix is too brittle, so it can not be applied to the skin in an efficient way. On the other hand, example 5 exhibits a matrix that is too soft, so it also is not appropriate for its use as a TDS. Regarding these facts, stability studies were not performed on examples 4 and 5.

Examples 1, 2 and 3 show differences regarding impurities formation during the accelerated stability studies. Examples 6 to 12 are compositions with different concentrations of HCl and TEC, showing that the protection exists within a concentration range of these compounds. The compositions which yield the best results are those of examples 2 and 6.

While the examples containing HCl or TEC do not avoid the formation of impurities, the combined presence of both compounds optimizes the stability of the formulation.
To avoid the formation of impurities means to avoid toxicity studies for the generated impurities, thus reducing the development costs and making the approval of the product easier.

A comparative bioavailability study was performed between patches comprised within the scope of the present invention and patches currently on the market (Exelon™) in 12 volunteers following good clinical practice (GCP).

The Exelon™ patch used was marketed in Argentina by Novartis. Exelon® Patch is indicated for the treatment of mild to moderate dementia of the Alzheimer's type and mild to moderate dementia associated with Parkinson's disease. The strength of the patch used was stated as 4.6 mg/24 hours, and it had a stated content of 9 mg of rivastigmine. The product's lot number was 204121.

The patch comprises a backing layer, a drug-containing acrylic matrix, a silicone adhesive matrix and an overlapping release liner, which is removed and discarded prior to use. Excipients within the formulation include acrylic copolymer, poly(butylmethacrylate, methyl-methacrylate), silicone adhesive applied to a flexible polymer backing film, silicone oil, and vitamin E.

Rivastigmine plasma levels were determined by a validated HPLC/MS-MS method. In the study 5.1 cm² patches with the formulation described in example 6 of table 3 and 5.0 cm² Exelon™ patches (9 mg/TDS) were used. The results are shown below in table 5 and Figure 1.

**Table 5**

| Time (h) | Plasma concentration by cm² (pg/ml.cm²) | | | |
|---|---|---|---|---|
| | Example 6 | | Exelon™ | |
| | Mean | Standard error | Mean | Standard error |
| 0 | 0 | 0 | 0 | 0 |
| 1 | 2,5 | 1,7 | 4 | 4 |
| 3 | 161,7 | 61,9 | 98.3 | 38.5 |
| 6 | 307,0 | 64,8 | 268.5 | 99.6 |
| 8 | 429,5 | 77,8 | 272.6 | 46.1 |
| 12 | 453,2 | 74,3 | 283.9 | 37.2 |
| 16 | 417,7 | 58,8 | 234.2 | 25.2 |
| 20 | 418,5 | 51,0 | 237.3 | 21.2 |
| 24 | 376,7 | 43,5 | 204.3 | 17.1 |

The plasma concentration profile (expressed by cm² of applied patch) shown in Figure 1 is similar to the one exhibited by the product Exelon™, although the Cmax and the AUC are a little higher for the formulation described in example 6 than for the product Exelon™. The observed difference can be corrected by decreasing the size of the patch described by this invention.

### 2) Selegiline (not according to the invention)

Devices with compositions as described in Table 5 were prepared. Duro-Tak® 87-4287 has a hydroxylic functionality and is not crosslinked. Duro-Tak® 87-4098 has no functionality and is not crosslinked.

**Table 5**

| Example | 1 | 2 |
|---|---|---|
| Selegiline base | 11.0 | 11.0 |
| Duro-Tak® 87-4287 | 71.9 | --- |
| Duro-Tak® 87-4098 | --- | 76.9 |
| Triethylcitrate | 2.0 | 2.0 |
| Ethylcellulose | 15.0 | 10.0 |
| Hydrochloric acid | 0.1 | 0.3 |

### 3) Rasagiline (not according to the invention)

Devices with compositions as described in Table 6 were prepared. Duro-Tak® 87-4287 has a hydroxylic functionality and is not crosslinked. Duro-Tak® 87-4098 has no functionality and is not crosslinked. Duro-Tak 87-2353 has a carboxylic functionality and is not crosslinked.

**Table 6**

| Example | 1 | 2 | 3 | 4 | 5 | 6 | 7 | 8 | 9 |
|---|---|---|---|---|---|---|---|---|---|
| Rasagiline base | 7.5 | 7.5 | 7.5 | 12.5 | 12.5 | 12.5 | 17.5 | 17.5 | 17.5 |
| Duro-Tak® 87-4287 | 81.5 | --- | --- | 76.0 | --- | --- | 70.0 | --- | --- |
| Duro-Tak® 87-4098 | --- | 91.5 | --- | --- | 86.0 | --- | --- | 80.0 | --- |
| Duro-Tak® 87-2353 | --- | --- | 76.5 | --- | --- | 71.0 | --- | --- | 65.0 |
| Triethylcitrate | 0.75 | 0.75 | 0.75 | 1.0 | 1.0 | 1.0 | 1.5 | 1.5 | 1.5 |
| Ethylcellulose | 10.0 | --- | 15.0 | 10.0 | --- | 15.0 | 10.0 | --- | 15.0 |
| Hydrochloric acid | 0.25 | 0.25 | 0.25 | 0.5 | 0.5 | 0.5 | 1.0 | 1.0 | 1.0 |

### 4) Nicotine (not according to the invention)

Devices with compositions as described in Table 7 were prepared. Duro-Tak 87-2353 has a carboxylic functionality and is not crosslinked. Duro-Tak® 87-2852 has a carboxylic functionality and is crosslinked.

**Table 7**

| Example | 1 | 2 | 3 | 4 | 5 | 6 |
|---|---|---|---|---|---|---|
| Nicotine base | 10.0 | 15.0 | 20.0 | 10.0 | 15.0 | 20.0 |
| Duro-Tak® 87-2353 | --- | --- | --- | 74.75 | 69.75 | 64.75 |
| Duro-Tak® 87-2852 | 84.75 | 79.75 | 74.75 | --- | --- | --- |
| Triethylcitrate | 5.0 | 7.5 | 10.0 | 5.0 | 7.5 | 10.0 |
| Ethylcellulose | --- | --- | --- | 10 | 15 | 20 |
| Hydrochloric acid | 0.25 | 0.5 | 0.75 | 0.25 | 0.5 | 0.75 |

### 5) Apomorphine (not according to the invention)

Devices with compositions as described in Table 8 were prepared. Duro-Tak 87-2353 has a carboxylic functionality and is not crosslinked. Duro-Tak® 87-2852 has a carboxylic functionality and is crosslinked.

**Table 8**

| Example | 1 | 2 | 3 | 4 | 5 | 6 |
|---|---|---|---|---|---|---|
| Apomorphine base | 5.0 | 7.5 | 10.0 | 5.0 | 7.5 | 10.0 |
| Duro-Tak® 87-2353 | --- | --- | --- | 86.85 | 77.2 | 68.0 |
| Duro-Tak® 87-2852 | 91.85 | 87.20 | 83.0 | --- | --- | --- |
| Triethylcitrate | 3.0 | 5.0 | 6.5 | 3.0 | 5.0 | 6.5 |
| Ethylcellulose | --- | --- | --- | 5.0 | 10.0 | 15.0 |
| Hydrochloric acid | 0.15 | 0.30 | 0.50 | 0.15 | 0.30 | 0.50 |

### 6) Agomelatine (not according to the invention)

Devices with compositions as described in Table 9 were prepared. Duro-Tak® 87-2287 has a hydroxylic functionality and is not crosslinked. Duro-Tak® 87-9088 has no functionality and is not crosslinked. Duro-Tak 87-2353 has a carboxylic functionality and is not crosslinked.

**Table 9**

| Example | 1 | 2 | 3 | 4 | 5 | 6 |
|---|---|---|---|---|---|---|
| Agomelatine base | 8.0 | 8.0 | 8.0 | 16.0 | 16.0 | 16.0 |
| Duro-Tak® 87-2353 | 77.8 | --- | --- | 60.6 | --- | --- |
| Duro-Tak® 87-2287 | --- | 77.8 | --- | --- | 60.6 | --- |
| Duro-Tak® 87-9088 | --- | --- | 88.8 | --- | --- | 75.6 |
| Triethylcitrate | 4.0 | 4.0 | 4.0 | 8.0 | 8.0 | 8.0 |
| Ethylcellulose | 10.0 | 10.0 | --- | 15.0 | 15.0 | --- |
| Hydrochloric acid | 0.2 | 0.2 | 0.2 | 0.4 | 0.4 | 0.4 |

### 7) Ropinirole (not according to the invention)

Devices with compositions as described in Table 10 were prepared. Duro-Tak® 87-2516 has a hydroxylic functionality and is crosslinked. Duro-Tak® 87-4098 has no functionality and is not crosslinked. Duro-Tak 87-2074 has both carboxylic and hydroxylic functionalities and is crosslinked.

**Table 10**

| Example | 1 | 2 | 3 | 4 | 5 | 6 |
|---|---|---|---|---|---|---|
| Ropinirole base | 15.0 | 15.0 | 15.0 | 25.0 | 25.0 | 25.0 |
| Duro-Tak® 87-2074 | 80.25 | --- | --- | 71.75 | --- | --- |
| Duro-Tak® 87-2516 | --- | 80.25 | --- | --- | 71.75 | --- |
| Duro-Tak® 87-4098 | --- | --- | 60.25 | --- | --- | 51.75 |
| Triethylcitrate | 4.0 | 4.0 | 4.0 | 7.0 | 7.0 | 7.0 |
| Ethylcellulose | --- | --- | 20.0 | --- | --- | 20.0 |
| Hydrochloric acid | 0.75 | 0.75 | 0.75 | 1.25 | 1.25 | 1.25 |

### 8) Asenapine (not according to the invention)

Devices with compositions as described in Table 11 were prepared.

**Table 11**

| Example | 1 | 2 | 3 | 4 | 5 | 6 |
|---|---|---|---|---|---|---|
| Asenapine base | 10.0 | 10.0 | 10.0 | 15.0 | 15.0 | 15.0 |
| Duro-Tak® 87-2287 | 76.75 | --- | --- | 56.5 | --- | --- |
| Duro-Tak® 87-4098 | --- | 76.75 | --- | --- | 56.5 | --- |
| Duro-Tak® 87-2353 | --- | --- | 76.75 | --- | --- | 56.5 |
| Triethylcitrate | 3.0 | 3.0 | 3.0 | 8.0 | 8.0 | 8.0 |
| Ethylcellulose | 10.0 | 10.0 | 10.0 | 20.0 | 20.0 | 20.0 |
| Hydrochloric acid | 0.25 | 0.25 | 0.25 | 0.50 | 0.50 | 0.50 |

## Claims

1. A device for the transdermal delivery of rivastigmine, **characterized by** comprising an adhesive matrix layer, a backing layer and a release or protective liner, wherein the adhesive matrix layer comprises rivastigmine, an amount of triethylcitrate of between 0.2% and 10%, and an amount of hydrochloric acid of between 0.05% and 5%.

2. The device described in claim 1, wherein triethylcitrate is present in an amount of between 1% and 5%.

3. The device described in claim 1, wherein hydrochloric acid is present in an amount of between 0.1% and 2%.

4. The device described in any of the previous claims, wherein the adhesive matrix comprises an adhesive polymer or copolymer that belongs to the group defined by polyacrylates, silicone polymers, polyisobutylenes and rubber block copolymers, such as those with styrene-isoprene-styrene of styrene-butyrene-styrene.

5. A device according to the previous claim, wherein the adhesive matrix comprises an adhesive polymer or copolymer belonging to the group formed by the polyacrylates.

6. The device described in the previous claim, wherein the adhesive matrix also comprises ethylcellulose in an amount of between 10% and 40%.

7. The device described the previous claim, wherein the adhesive matrix comprises an acrylate copolymer with hydroxylic or carboxylic functionality that is not crosslinked.

8. The device described in claim 1, wherein the amount of triethylcitrate is between 1% and 5%, the amount of HCl is between 0.1% and 2%, and the adhesive matrix also comprises ethylcellulose in an amount of between 10% and 40% and an acrylate copolymer with a hydroxylic functionality that is not crosslinked.

9. A device according to the previous claim, **characterized by** comprising 25% of rivastigmine base, 2% of triethylcitrate, 0.2% of hydrochloric acid and 25% of ethylcellulose.

10. A method for preparing a device for the transdermal delivery of rivastigmine comprising:
a) preparing a solution containing rivastigmine, a polymeric adhesive, triethylcitrate and hydrochloric acid;
b) pouring said solution on a release or protective liner so as to form a film that covers the liner;
c) drying said film at an appropriate temperature to obtain an adhesive matrix; and
d) attaching a backing layer to the adhesive matrix; wherein the adhesive matrix obtained comprises an amount of triethylcitrate of between 0.2% and 10%, and an amount of hydrochloric acid of between 0.05% and 5%.

11. The method described in the previous claim wherein the hydrochloric acid is added to the initial solution as a 1 N ethanolic solution before adding rivastigmine.

## Patentansprüche

1. Vorrichtung zur transdermalen Zufuhr von Rivastigmin, **dadurch gekennzeichnet, dass** sie eine Klebematrixschicht, eine Stützschicht und eine Freisetzungs- oder Schutzhülse umfasst, worin die Klebematrixschicht Rivastigmin, eine Menge von zwischen 0,2 % und 10 % Triethylcitrat und eine Menge von zwischen 0,05 % und 5 % Salzsäure umfasst.

2. Vorrichtung nach Anspruch 1, worin Triethylcitrat in einer Menge zwischen 1 % und 5 % vorliegt.

3. Vorrichtung nach Anspruch 1, worin Salzsäure in einer Menge zwischen 0,1 % und 2 % vorliegt.

4. Vorrichtung nach einem der vorangegangenen Ansprüche, worin die Klebematrix ein Klebepolymer oder -copolymer umfasst, das zu der durch Polyacrylate, Siliconpolymere, Polyisobutylene und Kautschukblockcopolymere definierten Gruppe gehört, wie z.B. jene mit Styrol-Isopren-Styrol von Styrol-Butyrol-Styrol.

5. Vorrichtung nach dem vorangegangenen Anspruch, worin die Klebematrix ein Klebepoylmer oder -copolymer umfasst, das zu der durch Polyacrylate ausgebildeten Gruppe gehört.

6. Vorrichtung nach dem vorangegangenen Anspruch, worin die Klebematrix auch Ethylcellulose in einer Menge zwischen 10 % und 40 % umfasst.

7. Vorrichtung nach dem vorangegangenen Anspruch, worin die Klebematrix ein Acrylatcopolymer mit Hydroxy- oder Carboxy-Funktionalität umfasst, das nicht vernetzt ist.

8. Vorrichtung nach Anspruch 1, worin die Menge Triethylcitrat zwischen 1 % und 5 % liegt, die Menge HCl zwischen 0,1 % und 2 % liegt und die Klebematrix auch Ethylcellulose in einer Menge zwischen 10 % und 40 % und ein Acrylatcopolymer mit einer Hydroxyl-Funktionalität umfasst, das nicht vernetzt ist.

9. Vorrichtung nach dem vorangegangenen Anspruch, **dadurch gekennzeichnet, dass** sie 25 % Rivastigminbase, 2 % Triethylcitrat, 0,2 % Salzsäure und 25 % Ethylcellulose umfasst.

10. Verfahren zur Herstellung einer Vorrichtung für die transdermale Zufuhr von Rivastigmin, das Folgendes umfasst:
a) das Herstellen einer Rivastigmin, einen polymeren Klebstoff, Triethylcitrat und Salzsäure enthaltenden Lösung;
b) das Gießen der Lösung auf eine Freisetzungs- oder Schutzhülse, um so einen Film auszubilden, der die Hülse überdeckt;
c) das Trocknen des Films bei einer angemessenen Temperatur, um eine Klebematrix zu erhalten; und
d) das Anbringen einer Stützschicht an der Klebematrix;
worin die erhaltene Klebematrix eine Menge von zwischen 0,2 % und 10 % Triethylcitrat und eine Menge von zwischen 0,05 % und 5 % Salzsäure umfasst.

11. Verfahren nach dem vorangegangenen Anspruch, worin die Salzsäure vor dem Zusetzen von Rivastigmin als 1 N Ethanol-Lösung zu der Anfangslösung zugesetzt wird.

## Revendications

1. Dispositif d'administration transdermique de rivastigmine, **caractérisé par le fait qu'**il comprend une couche de matrice adhésive, une couche de support et un revêtement anti-adhésif ou protecteur, dans lequel la couche de matrice adhésive comprend de la rivastigmine, une quantité de triéthylcitrate située entre 0,2 % et 10 %, et une quantité d'acide chlorhydrique située entre 0,05 % et 5 %.

2. Dispositif décrit dans la revendication 1, dans lequel le triéthylcitrate est présent en une quantité située entre 1 % et 5 %.

3. Dispositif décrit dans la revendication 1, dans lequel l'acide chlorhydrique est présent en une quantité située entre 0,1 % et 2 %.

4. Dispositif décrit dans l'une quelconque des revendications précédentes, dans lequel la matrice adhésive comprend un polymère ou un copolymère adhésif qui appartient au groupe défini par les polyacrylates, les polymères de silicone, les polyisobutylènes et les copolymères séquencés de type caoutchouc, tels que ceux comportant du styrène-isoprène-styrène de styrène-butyrène-styrène.

5. Dispositif selon la revendication précédente, dans lequel la matrice adhésive comprend un polymère ou un copolymère adhésif appartenant au groupe formé par les polyacrylates.

6. Dispositif décrit dans la revendication précédente, dans lequel la matrice adhésive comprend également de l'éthylcellulose en une quantité située entre 10 % et 40 %.

7. Dispositif décrit dans la revendication précédente, dans lequel la matrice adhésive comprend un copolymère d'acrylate comportant une fonctionnalité hydroxylique ou carboxylique qui n'est pas réticulé.

8. Dispositif décrit dans la revendication 1, dans lequel la quantité de triéthylcitrate se situe entre 1 % et 5 %, la quantité de HCl se situe entre 0,1 % et 2 %, et la matrice adhésive comprend également de l'éthylcellulose en une quantité située entre 10 % et 40 % et un copolymère d'acrylate comportant une fonctionnalité hydroxylique qui n'est pas réticulé.

9. Dispositif selon la revendication précédente, **caractérisé par le fait qu'**il comprend 25 % de base de rivastigmine, 2 % de triéthylcitrate, 0,2 % d'acide chlorhydrique et 25 % d'éthylcellulose.

10. Procédé de préparation d'un dispositif d'administration transdermique de rivastigmine comprenant les étapes consistant à :
a) préparer une solution contenant de la rivastigmine, un adhésif polymère, du triéthylcitrate et de l'acide chlorhydrique ;
b) verser ladite solution sur un revêtement anti-adhésif ou protecteur de manière à former un film qui recouvre le revêtement ;
c) sécher ledit film à une température appropriée pour obtenir une matrice adhésive ; et
d) fixer une couche de support sur la matrice adhésive ; dans lequel la matrice adhésive obtenue comprend une quantité de triéthylcitrate située entre 0,2 % et 10 %, et une quantité d'acide chlorhydrique située entre 0,05 % et 5 %.

11. Procédé décrit dans la revendication précédente dans lequel l'acide chlorhydrique est ajouté à la solution initiale sous la forme d'une solution éthanolique 1 N avant l'ajout de rivastigmine.
